# EUROPEAN PATENT SPECIFICATION

(11) **EP 4 178 354 B1**
(45) Date of publication and mention of the grant of the patent: **06.05.2026**
(21) Application number: 21751836.4
(22) Date of filing: 13.07.2021
(51) Int. Cl.: A01N 25/00, A01N 33/12, A01N 37/16, A01N 59/00, A01P 1/00, A61L 2/00, A61L 2/28, G01N 31/22, C11D 3/48, C09B 67/30, C11D 3/40, C11D 17/04

(54) **TWO-PART DISINFECTANT SYSTEM COMPRISING A COLOUR INDICATOR**
ZWEITEILIGES DESINFEKTIONSSYSTEM MIT FARBINDIKATOR
SYSTÈME DÉSINFECTANT EN DEUX PARTIES COMPRENANT UN INDICATEUR DE COULEUR

(30) Priority: 13.07.2020 GB 202010736
(43) Date of publication of application: 17.05.2023
(73) Proprietor: Tristel PLC, Snailwell, Newmarket CB8 7NY (GB)
(72) Inventor: BRAND, Thomas, Newmarket CB8 7NY (GB)
(74) Representative: Dummett Copp LLP
(86) International application number: PCT/GB2021/051793
(87) International publication number: WO 2022/013543

(56) References cited:
- WO-A1-02/091832
- WO-A1-2006/113166
- CN-U- 205 580 978
- GB-A- 2 413 765
- JP-A- 2016 113 354
- US-A1- 2009 176 673
- HIROSHI MATSUFUJI ET AL: "Stability to Light, Heat, and Hydrogen Peroxide at Different pH Values and DPPH Radical Scavenging Activity of Acylated Anthocyanins from Red Radish Extract", JOURNAL OF AGRICULTURAL AND FOOD CHEMISTRY, vol. 55, no. 9, 1 May 2007 (2007-05-01), US, pages 3692 - 3701, XP055299942, ISSN: 0021-8561, DOI: 10.1021/jf063598o
- �ZKAN MEHMET ET AL: "EFFECT OF HYDROGEN PEROXIDE ON SOUR CHERRY ANTHOCYANINS", JOURNAL OF FOOD QUALITY, 1 August 2000 (2000-08-01), Oxford, UK, pages 421 - 428, XP055928687, Retrieved from the Internet <URL:https://cyberleninka.org/article/n/239952.pdf> [retrieved on 20220608], DOI: 10.1111/j.1745-4557.2000.tb00568.x

## Description

### BACKGROUND

### a. Field of the Invention

The present invention relates to a disinfectant system, notably to a system for preparing chlorine dioxide using a two-part chemistry. The invention is particularly for use in disinfecting medical devices and surfaces, notably surfaces in clinical environments, but it is not limited to these uses.

### b. Related Art

Two-part disinfectant systems which produce chlorine dioxide when mixed are known. Such systems typically include a chlorite and an acid, or a chlorate, a reducing agent and an acid.

WO 2005/011756 discloses a two-part disinfecting system (shown in Figure 1). The disinfecting system 6 comprises a first part having a first reagent in a carrier medium and a second part which is miscible with the first part and which comprises a second reagent in a carrier medium. The first reagent and the second reagent react when mixed to provide a disinfecting composition. The first part is contained in a pump dispenser 2 whereby it may be dispensed as a fluid, preferably as a foam, and the second part is absorbed or impregnated in at least one fabric member in a sealed container 4. To prepare a disinfecting wipe, a user removes an impregnated wipe from the container, and applies a portion of foam from the sprayer to the wipe. To facilitate mixing of the reagents in the foam and the wipe, the user may fold the wipe in half and crush or rub the folded wipe before opening it out.

WO 2005/107823 describes a system (shown in Figure 2) suitable for the reprocessing of non-lumened medical devices using a manual three-wipe disinfection process. An example system includes a box 10 containing sachets 8 of pre-clean wipes, a disinfecting system 6 as discussed above, and a box 14 containing sachets 12 of sterile rinse wipes. The pre clean wipe is used to wipe an item such as an endoscope which is to be decontaminated. The two-part disinfecting system 6 (combination of a wipe and activator foam) is used for sterilising or disinfecting the item and the sterile rinse wipe is used to remove any chemical residue. All disinfection details can be recorded in an accompanying audit trail book to allow full procedural traceability.

WO 2006/079822 A1 describes another disinfecting system. In this case the first and second reagents are each carried in aqueous media to which foam promoters are added, so that both the first and second parts of the system are dispensed as first and second foams respectively. The first and second foams are mixed to generate the disinfecting composition, which may then be applied to an item or surface to be disinfected directly or with a wipe.

WO 02/091832 A1 describes another two-part disinfecting system, where the two parts are adapted to be mixed to yield an aqueous disinfecting composition. The first part comprises a chlorite and the second part comprises an acid. The composition further comprises an alpha olefin sulfonate to suppression chlorine dioxide formation.

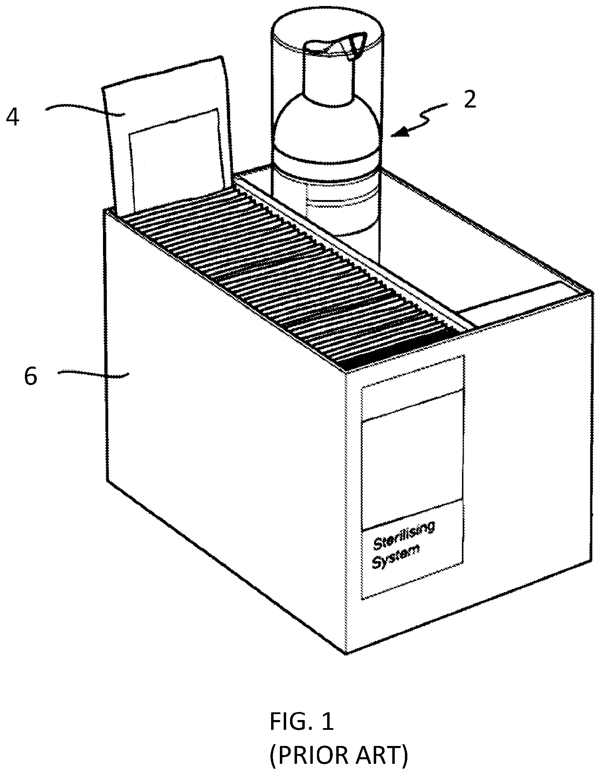

GB 2 413 765A discloses a two-part disinfectant system, which corresponds to the system described in present figure 1. The dye used is a pH-sensitive dye, different from the dyes used in the present invention.

JP 2016 113354 A discloses a two-part disinfectant system, in which the indicator, different from the dyes of the present system, is oxidized by chlorine dioxide.

To ensure full effectiveness of a disinfectant wipe or other chlorine dioxide disinfecting system, it is desirable to ensure that chlorine dioxide has been generated and that the action of generating chlorine dioxide can be verified by the end user.

It has been proposed to include in one of the components a pH-sensitive indicator which changes colour or becomes coloured when adequate mixing has occurred. A problem with this approach is that pH may not change much, or a change in pH may not reliably correlate with generation of sufficient chlorine dioxide.

As the medical industry develops there are pressures to move towards automated disinfection systems that are perceived to provide additional assurances that the disinfection process has been successfully completed. The primary arguments in favour of these systems are that they eliminate or reduce the probability of user error and can provide a digital ticket at the end of a machine cycle.

Currently available technologies include test strips, odour detection, titrations and spectrophotometry. Although all able to determine chlorine dioxide concentration they are limited by accuracy of measurement (with some methods semiquantitative), the requirement for a laboratory facility and the impingement on the natural process flow of device reprocessing.

### SUMMARY OF THE INVENTION

Aspects of the invention are specified in the independent claims. Preferred features are specified in the dependent claims.

By incorporating a suitable dyestuff in one of the parts of the system, it is possible to verify, either by eye or by opto-electronic means, that chlorine dioxide has been produced at an efficacious level in the combined parts prior to use for sterilisation or disinfection. It is also possible to verify that chlorine dioxide has been produced in the whole of the medium comprising the combined parts, by checking whether the colour change has occurred spatially uniformly throughout the medium.

Suitable dyestuffs are those that oxidise in the presence of chlorine dioxide to produce a visible colour change upon mixing of the first reagent with the second reagent, but that do not exhibit the same colour change upon exposure to a disinfecting composition comprising hydrogen peroxide and/or peracetic acid. Preferably, the dyestuff also does not exhibit the same colour change upon exposure to disinfecting compositions including quaternary ammonium compounds and/or triamines.

The dyestuffs used in embodiments of the invention are therefore selective for chlorine dioxide, meaning that the colour change does not occur in the presence of other commonly-used high-level disinfectants or sterilants. In this way, the present invention provides a safeguard against incorrect use of the disinfectant system, for example in scenarios where one part of the system could be mistakenly substituted with one or more other disinfectant products.

According to the invention, the dyestuff comprises an anthocyanin dyestuff selected from the group consisting of: black carrot extract, purple carrot extract, haskapa berry extract, and blackcurrant extract; an anthocyanidin dyestuff selected from the group consisting of bilberry extract and blue pea extract (clitoria ternatea); or a betanin dyestuff selected from the group consisting of red beetroot powder and beetroot juice concentrate. In a particularly preferred embodiment, the dyestuff comprises Black Carrot Extract (an anthocyanin dyestuff, also referred to as Antho Black Carrot Extract or AnthoCarrot).

The dyestuff preferably changes from coloured to colourless in the presence of chlorine dioxide. In such cases, the part of the system containing the dyestuff preferably exhibits a distinctive pre-exposure colour of the dyestuff, which is observed to disappear after efficacious mixing of the two parts.

The first reagent may comprise a metal chlorite and the second reagent may comprise an acid. The dyestuff may be provided in either the first part, or the second part, or even in both parts.

The first part and the second part of the disinfectant system can each be of any form, subject to compatibility with one another. In general, the first part could for example be any one of a liquid, a foam or a powder or may be impregnated into or otherwise carried on a wipe. The second part could also for example be any one of a liquid, a foam or a powder or may be absorbed or impregnated into or otherwise carried on a wipe. The disinfecting composition may be ready to use, with no dilution required after mixing of the two parts, or may be concentrated for subsequent dilution after mixing to provide a suitable concentration for use.

In one embodiment, the first part is contained in a dispenser whereby it may be dispensed as a fluid, in particular as a liquid or a foam, and the second part is absorbed or impregnated in at least one fabric wipe. In such cases, the second part may comprise the dyestuff, so that the colour change can be most readily observed when the first part is applied to the wipe.

It has been found that, in applications in which the part containing the dyestuff is carried by a wipe, the colour change that occurs upon exposure to chlorine dioxide may be less discernible compared to applications in which the part containing the dyestuff is provided in other forms, such as a liquid. Accordingly, for applications including wipes, it may be preferable that the dyestuff has a strong, distinctive colour that contrasts with the base colour of the wipe (usually white) and/or the yellowish colour of chlorine dioxide. Anthocyanin, anthocyanidin and betanin dyes typically have characteristic deep colours in the red/purple/blue region of the spectrum and therefore provide a clearly distinguishable colour change particularly in applications in which the part containing the dyestuff is carried by a wipe. Black carrot extract, for example, has been found to be particularly effective in providing a distinctive pre-reaction colour when applied to a wipe.

In another example, the first part and/or the second part comprise foams. Again, in this case, it may be preferable that the dyestuff has a strong, distinctive colour that is readily visible in the foam, which usually has a white appearance in the absence of a dyestuff due to light scattering effects. In this connection, the characteristic deep colours associated with anthocyanin, anthocyanidin and betanin dyes may be of particular benefit.

The first part or the second part respectively may comprise between about 0.01% and about 2% dyestuff. Preferably, after mixing of the first reagent with the second reagent, substantially all of the dyestuff is oxidised by the resulting chlorine dioxide.

Also disclosed is a method of verifying the production of a chlorine dioxide disinfecting composition using a disinfectant system as described above. The method comprises mixing the first part and the second part then, during mixing of the first part and the second part, observing said colour change, and determining that the chlorine dioxide disinfecting composition has been produced when said colour change is complete and spatially uniform. The colour change may be observed by eye, or by a suitable opto-electrical system. A suitable machine vision system may be used to perform the observing and determining steps.

For disinfectant systems including a wipe, a method of determining whether the wipe contains chlorine dioxide comprises illuminating at least one surface region of the fabric wipe with at least one wavelength of light, determining an intensity value by measuring the intensity of the at least one wavelength of light reflected from at least one surface region of the fabric wipe, said wavelength corresponding to a wavelength absorbed by the dyestuff, comparing the intensity value with a preset threshold value, and signalling that the wipe contains sufficient chlorine dioxide if the intensity value is at or above the threshold value, or signalling that the wipe contains insufficient chlorine dioxide if the intensity value is below the threshold value. A corresponding apparatus comprises a device for measuring, from at least one surface region of a wipe, an intensity value of at least one wavelength of light corresponding to a wavelength absorbed by the dyestuff, a comparator device for comparing the intensity value with a preset threshold value, and a signalling device for signalling that a wipe contains sufficient chlorine dioxide if the intensity value is at or above the threshold value, or signalling that the wipe contains insufficient chlorine dioxide if the intensity value is below the threshold value.

Also disclosed is a disinfectant system comprising a first part comprising a first reagent in a carrier medium; and a second part which is miscible with the first part and which comprises a second reagent in a carrier medium, wherein the first reagent and the second reagent will react when the first and second parts are mixed to provide a chlorine dioxide disinfecting composition. The first part or the second part further comprises an anthocyanin dyestuff, an anthocyanidin dyestuff, or a betanin dyestuff.

Preferred and/or optional features of each aspect and embodiment of the invention may also be used, alone or in appropriate combination, in the other aspects and embodiments also.

### BRIEF DESCRIPTION OF THE DRAWINGS

The invention will now be further described, by way of example only, with reference to the following drawings, in which:
Figures 1 and 2 show prior art disinfectant systems;
Figure 3 shows a disinfectant system in accordance with an embodiment of the present invention;
Figure 4 shows absorbance measurements as a function of wavelength for an anthocyanin dyestuff in one part of a two-part disinfectant system before and after activation with the other part of the disinfectant system;
Figure 5 shows chlorine dioxide generation as a function of time for a disinfectant system including an anthocyanin dyestuff and for a disinfectant system not including a dyestuff;
Figure 6 shows comparative examples of fabric wipes treated with different disinfecting compositions;
Figure 7 and shows absorbance measurements as a function of wavelength for an anthocyanin dyestuff solution upon exposure to different disinfecting compositions; and
Figure 8 is a schematic view of apparatus not in accordance with the invention but useful for its understanding.

### DETAILED DESCRIPTION

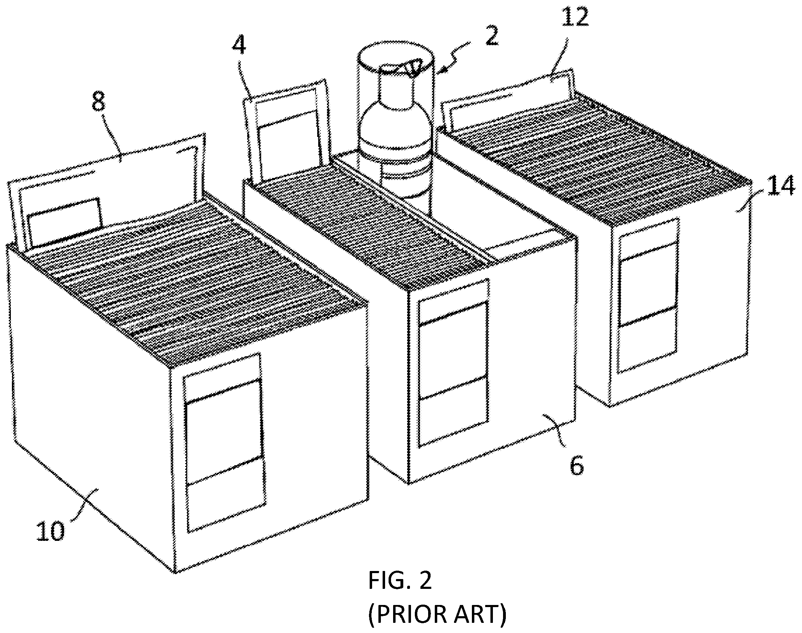

The prior art systems illustrated in Figures 1 and 2 are suitable for use in the present invention, but with the difference that, in a first embodiment, the second part further comprises an anthocyanin dyestuff.

In the present example, the first part comprises less than 1% sodium chlorite, and less than 2.5% amphoteric surfactant. The remainder is deionised water. In this specification, all parts are by weight unless otherwise indicated. Operation of the pump trigger dispenses the first part as a foam.

The wipes 16 are impregnated with an aqueous acid solution (second part). In this example, the acid solution comprises 1-5% citric acid and 1% Antho Black Carrot Extract as the anthocyanin dyestuff. The remainder is deionised water.

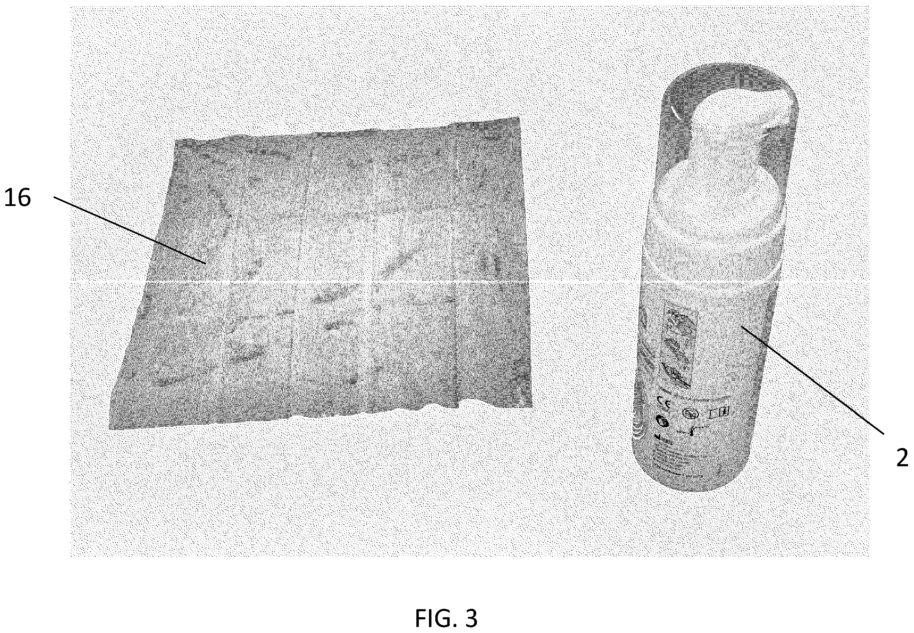

The anthocyanin dyestuff provides the fabric wipe 16 (Figure 3) with a characteristic colour. In this embodiment, the wipe 16 has a pinkish-red colour before the first part is added. We have found that, in the presence of chlorine dioxide, the anthocyanin dyestuff is readily oxidised to a non-coloured substance. This results in the wipe 16 losing its characteristic colour. The system provides a positive indication when chlorine dioxide has been generated and the action of generating the chlorine dioxide can be verified by the end user.

Use of a stable, selective dyestuff allows for the verification of not only the presence of chlorine dioxide but additionally the correct level of chlorine dioxide to ensure efficacy. The dyestuff also provides an environmental risk mitigator in the event of using the product outside of its recommended use temperature. The rationale is that the rate of chlorine dioxide generation is slower at colder temperatures and faster at higher temperatures. The rate of dye oxidation will be proportional to the level of chlorine dioxide generated.

Various dyestuffs were investigated for the ability to provide selectivity towards chlorine dioxide. In addition, the safety profile of each dye required assessment to ensure that during the oxidation process no harmful byproducts were formed which would have the potential to be detrimental to patient safety.

Potassium iodide, for example, exhibits a detectable colour change upon oxidation in the presence of chlorine dioxide, but also exhibits the same behaviour with other common oxidising disinfectants, such as hydrogen peroxide. Potassium iodide is therefore non-selective for chlorine dioxide and is not suitable for use in the present invention. Metal-based pigments are generally not suitable, as they are not readily oxidised by chlorine dioxide.

Research led to the finding that anthocyanin-based dyes, anthocyanidin-based dyes and betanin-based dyes are suitable for use in chlorine dioxide disinfectant systems because of ease of oxidation, selectivity and safety.

Anthocyanins, such as Antho Black Carrot Extract (AnthoCarrot), are a family of naturally derived pigments which are often responsible for the red-blue colours observed in fruits and vegetables. These compounds are readily found in food, being present in much of our produce as well as being used as natural dyes and food additives. Anthocyanins for food use are referred to by the E-number E163 and include E163(ii) Grape skin extract (Enociania, Eno), E163(iii) Blackcurrant extract, E163(iv) Purple corn colour, E163(v) Red cabbage colour, E163 (vi) Black carrot extract, E163 (vii) Purple sweet potato colour, E163 (viii) Red radish colour, E163(ix) Elderberry colour and E163(x) Hibiscus colour.

Anthocyanins have no reported toxicological information or warnings and are generally considered safe for use.

Anthocyanin (AnthoCarrot) is red when incorporated into a phase two liquid solution (i.e. the aqueous acid second part of the disinfectant system) and is oxidised to colourless when activated with a phase one foam (i.e. a foam containing the sodium chlorite-containing first part). In order to assess the degree of oxidation of anthocyanin, post activation, spectrophotometric analysis of activated (mixed) and non-activated (before mixing) phase two liquid solution with 0.5% anthocyanin (AnthoCarrot) was conducted.

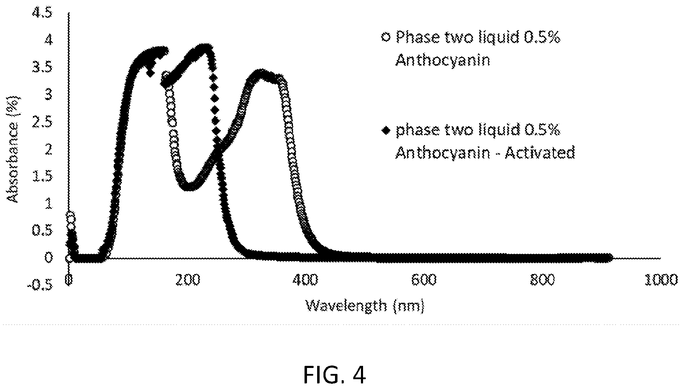

Samples were activated with a 1:3 addition of phase one foam solution to phase two liquid solution and allowed to reach peak chlorine dioxide generation before testing (2 minutes). Figure 4 shows the spectroscopic results, which shows that all anthocyanin is broken down by the generated chlorine dioxide. This can be best observed by the absence of the peak at 350nm in the activated sample versus the non-activated sample, when maintaining equal anthocyanin starting concentrations.

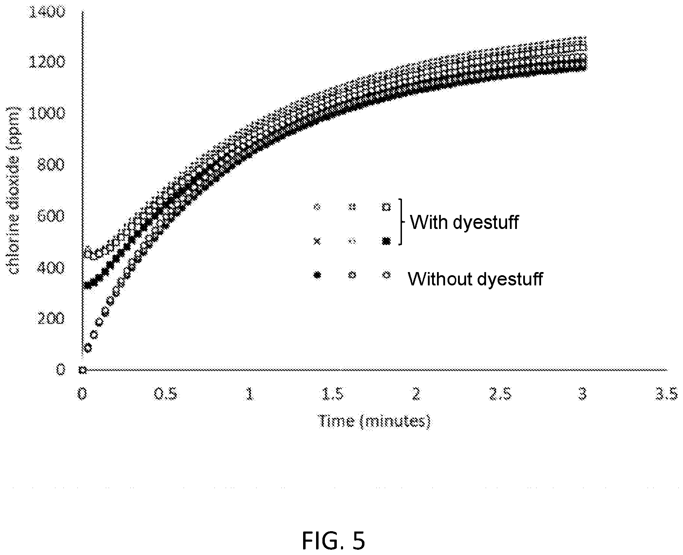

Several chlorine dioxide generation tests were conducted to ensure that, although oxidation of the dyestuff consumes some chlorine dioxide, overall chlorine dioxide generation is still reaching expected levels. Figure 5 shows chloride dioxide generation profiles for multiple samples of the foam-liquid system described above with and without the dyestuff. The graph of Figure 5 shows that, during the initial reaction of the dyestuff with chlorine dioxide, slight interference is observed, however after about 30 seconds curve profiles and peak levels, within experimental error, are substantially identical.

Confirmatory microbiological testing was also conducted which shows efficacy is maintained on inclusion of the selective dyestuff.

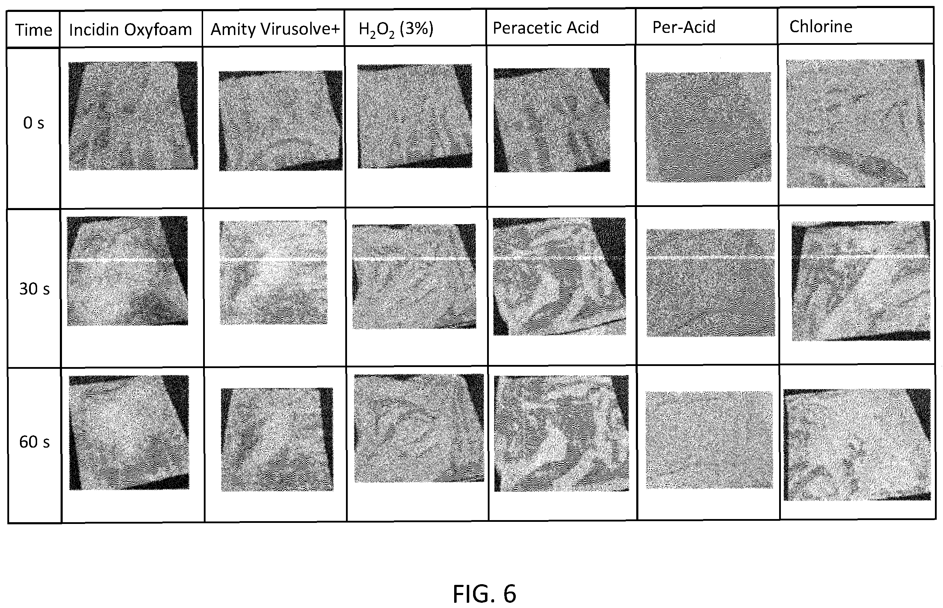

Dye selectivity testing was conducted to test whether the anthocyanin dye would react or degrade in the presence of other common use high level disinfectants and oxidisers, including hydrogen peroxide, peracetic acid, and chlorine. Tests were conducted on wipes impregnated with 9 ml of 1% AnthoCarrot base, with 3 ml of each product (equivalent to a single dose of the first part from the pump 2). The wipes were then scrunched by hand for 15 s with pictures taken at time points 0, 30 and 60s. The wipes are shown in Figure 6, in which light contrast areas correspond to the characteristic pink-red colour of the AnthoCarrot dye and dark contrast areas correspond to wet regions of the wipes contacting the work surface. The results are summarised in Table 1.

**Table 1**

| **Test product** | **Description** | **Colour change after 60s** |
|---|---|---|
| Incidin Oxyfoam | hydrogen peroxide-based disinfectant | none |
| Amity Virusolve + | bis (3-aminopropyl) dodecylamine with didecyldimethyl ammonium chloride | none |
| H₂O₂ | hydrogen peroxide 3% | none |
| Peracetic acid | 10,000 ppm stabilised with hydrogen peroxide | none |
| Per-acid | 10,000 ppm stabilised with hydrogen peroxide | none |
| Chlorine | 1000 ppm | incomplete colour loss |

As can be seen, only chlorine (1000 ppm) influenced the dye. No other high level disinfectant or strong oxidiser fully degraded the colour in the wipes. Chlorine successfully removed much of the dye colouration, however it was not able to fully degrade it, with some spotting remaining even at 60 s. Furthermore, the wipe itself was affected by chlorine, with it turning to an off-white colour rather than its original colour, as the wipes do when exposed to chlorine dioxide. Based on this it can be safely said that the anthocyanin dye has a high specific selectivity for chlorine dioxide and can be used as a measure of wipe activation.

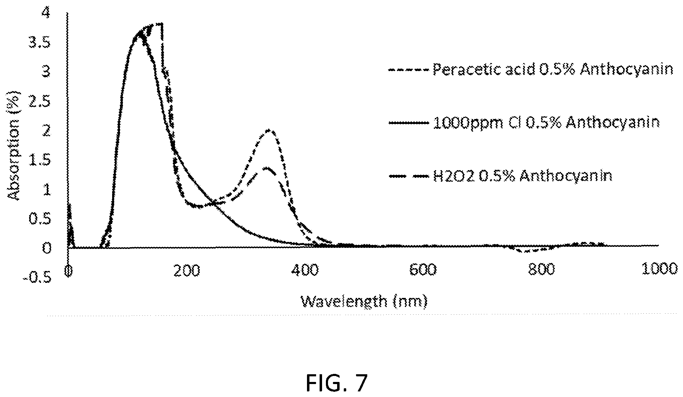

In a further selectivity test, 0.5% anthocyanin (AnthoCarrot) was added to common oxidisers in liquid form and the resulting mixture spectroscopically analysed. The results are shown in Figure 7, and show that peracetic acid and hydrogen peroxide were unable to fully oxidise the dye with colour still observed. Chlorine mostly decomposed the dye, but a precipitate formed.

Various other dyes have been tested to assess suitability for use in the present invention. The primary requirements for a suitable dyestuff are that it is stable in at least one of the two parts of the disinfectant system, was readily oxidised in the presence of chlorine dioxide and was unstable or not oxidised in other leading oxidising compounds.

Samples of dyes were tested on liquid and wipe variants of the second part of the system for stability and chlorine dioxide generated from the combination of the second part with samples of liquid and foam versions of the first part, and in addition tested against the below oxidisers/disinfectants which are commonly used:
Hydrogen peroxide (30,000ppm)
Hydrogen peroxide spray - with surfactant (commercial product - Ecolab Oxifoam)
Peracetic acid 10,000ppm
Peracetic acid/hydrogen peroxide combination (10,000ppm)
Chlorine 1,000ppm
Triamine/quaternary ammonium disinfectant (Amity Virosol +)

The following types of dyestuffs were tested and identified as suitable for selective oxidation by chlorine dioxide and stability prior to use. None of the dyes produced an identical outcome result when used with the above disinfectants:
Anthocyanins, including black carrot extract (e.g. AnthoCarrot L-WS E163); purple carrot powder, haskapa berry extract, E163 food colouring additives; blackcurrant extract;
Anthocyanidins, including bilberry extract and clitoria ternatea (blue pea extract);
Betanins, including red beetroot powder, E162 beetroot powder and beetroot juice concentrate.

Several other dyestuffs were found to be less suitable for use, compared to anthocyanin, anthocyanidin and betanin dyestuffs. For example:
Carmosine (E122) and allura red (E129) are readily oxidised by chlorine dioxide but have the potential to form harmful by-products if subjected to extremes of heat;
Tartrazine (E102, CI 19140 FD&C Yellow) was found to oxidise in the presence of oxidising disinfectants other than chlorine dioxide;
Chromium oxide did not fully oxidise in the presence of chlorine dioxide and produced a precipitate on reaction;
Ponceau (E124) did not oxidise when exposed to chlorine dioxide at levels up to 2000 ppm;
Indigo carmine has the potential to form harmful by-products;
Xanthene dyes have the potential to form harmful degradation products; and
Brilliant Blue FCF (E133, Food Blue 2 CI 42090) was found to oxidise in the presence of oxidising disinfectants other than chlorine dioxide.

A further benefit of the disinfectant system of the present invention is that the use of a chlorine dioxide-selective dyestuff allows for confirmation not just that chlorine dioxide has been produced, but that the correct level of chlorine dioxide has been generated. The rate at which the chosen dyestuff is oxidised is directly proportional to the rate of inclusion. It is therefore possible to tailor the inclusion rate (i.e. the amount of dyestuff added) to ensure that total removal of colour occurs once a certain chlorine dioxide level has been reached.

It is known that chlorine dioxide rate of generation is directly proportional to temperature (see, for example, Mo et at., "Kinetics of the Preparation of Chlorine dioxide by Sodium Chlorite and Hydrochloric Acid at Low Concentration", Chemical Engineering Transactions (46) 49-54 2015). Higher temperatures result in faster rates of generation. As the rate of dye oxidation is directly proportional to the rate of generation, the inclusion of a selective chlorine dioxide dye indicator allows for the mitigation of temperature on the rate of generation in use. Accordingly, regardless of the temperature at which the system is being used, it can be relied upon that until all dye colour has visibly disappeared the desired chlorine dioxide level has not been reached.

Testing was conducted to show that the inclusion of an anthocyanin dyestuff provided positive confirmation of the correct level of chlorine dioxide. Testing was conducted using the AnthoCarrot dye and two other comparative examples with solutions reacted at various temperatures, as set out in Table 2. When all dyestuff was oxidised, spectrophotometry analysis confirmed that intended levels of chlorine dioxide had been generated. Analysing chlorine dioxide levels before all dye had oxidised showed sub optimal levels of chlorine dioxide. Each dye tested was used in a different chlorine dioxide formulation with a different intended final concentration to show that the verification effect was observed across multiple dye stuffs and products.

**Table 2: time to total dye oxidation and desired chlorine dioxide generation (average of three replicates)**

| | **Temperature** | | |
|---|---|---|---|
| **Dyestuff** | **4°C** | **20°C** | **60°C** |
| Anthocyanin | 180 seconds | 83 seconds | 34 seconds |
| Food Blue 2 CI 42090 / CI 19140 FDC yellow dye | 92 seconds | 31 seconds | 3 seconds |
| Xanthene dye (red) | 80 seconds | 38 seconds | 5 seconds |

These results show that various dyestuffs, including anthocyanin dyestuffs suitable for use in the present invention, can effectively confirm the presence of a desired level of chlorine dioxide by exhibiting total oxidation, regardless of temperature. By increasing or decreasing the dye inclusion rate, the concentration of chlorine dioxide at which the dye is fully oxidised can be suitably selected.

Using a camera and appropriate software, or other suitable apparatus, it is possible to accurately determine the presence or absence of the dye pigment. This allows robust verification that active chlorine dioxide has been generated.

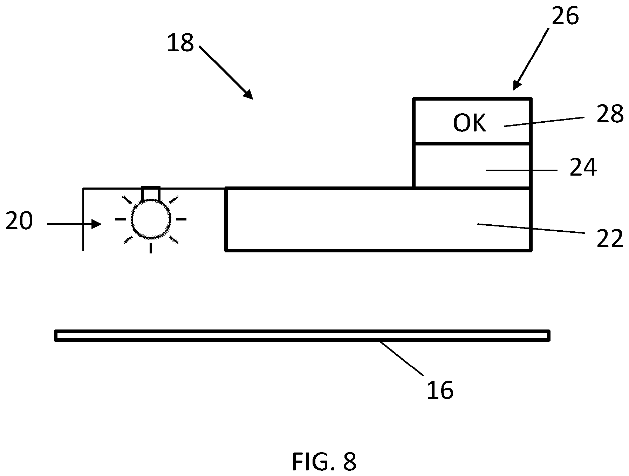

Referring now to Figure 8, a schematic apparatus 18 for determining whether a wipe 16 contains sufficient chlorine dioxide is illustrated. The apparatus 18 includes a lamp 20 for illuminating at least one surface region of the fabric wipe 16 with at least one wavelength of light corresponding to a wavelength absorbed by the selective dyestuff. In the present example, Antho Black Carrot Extract absorbs light in the range about 450 to about 560 nm, with a peak at about 530 nm (green). The apparatus 18 includes a device 22 for measuring the intensity of the at least one wavelength of light, and a comparator device 24 for comparing the intensity value with a preset threshold value. A signalling device 26 signals that the wipe contains sufficient chlorine dioxide if the intensity value is at or above the threshold value (indicating that the dyestuff is not absorbing the light). If the intensity value is below the threshold value, the signalling device 26 signals that the wipe 16 contains insufficient chlorine dioxide (the dyestuff is absorbing the light). In this example, the signalling device includes a display 28 that provides a visual indicator. It will be understood that other signals may be used, including sounds or coloured lights, and that signals may additionally or alternatively be logged digitally on a PC or other suitable device.

The step of determining the intensity value may comprise measuring the intensity of at least one wavelength of light reflected from a plurality of surface regions of the fabric wipe and calculating the intensity value as the mean average of each measurement. To facilitate this, the apparatus 18 may further comprise a component for calculating the intensity value as the mean average of a plurality of intensity measurements.

The apparatus may be arranged to measure the intensity of multiple wavelengths of light and/or a range of wavelengths of light. The wavelength or wavelengths selected should preferably correspond to a wavelength that is strongly absorbed by the dye before activation and that is not absorbed after activation, and preferably should avoid any interference from chlorine dioxide, which exhibits absorption at around 360nm and around 445nm. For Antho Black Carrot Extract, the selected wavelength may be in the range of between around 500 nm and around 560 nm, and is preferably around 530 nm.

As an alternative to spectroscopic analysis, it is also possible to use a machine vision system in which image analysis techniques are used to discern the pre- and post-reaction colours to determine when sufficient chlorine dioxide has been produced.

The term "fluid" is used herein to include liquids, foams, sprays, pastes, aerosols, powders, sols and gels. It is particularly preferred that the first part is dispensed as a foam or a spray to facilitate its coverage of a desired area of the wipe. The term "chlorine dioxide disinfecting composition" is used to refer to disinfecting compositions in which the active agent is chlorine dioxide.

While the above-described examples relate to a disinfectant system comprising a foam activator (providing the first part) and a fabric wipe (providing the second part), with the dyestuff included in the second part, this is merely one example. Table 3 describes examples of possible delivery forms for the first part, including sodium chlorite, and Table 4 describes examples of possible delivery forms for the second part, including an acid.

**Table 3: First part examples**

| **Form** | **Description** |
|---|---|
| Powder | A product containing powdered sodium chlorite as the chlorine dioxide releasing agent with a strength of 5-15% (nominal 10%), and a flow agent to assist with maintaining powder properties and reducing clumping. |
| Liquid | A product containing liquid sodium chlorite as the chlorine dioxide releasing agent with a strength of 0.25% to 10% (0.5-4.0% nominal) and a diluent (preferably water, alternatively glycols, ethers, surfactants or any combination of these). |
| Foam | A product utilising a liquid formulation containing liquid sodium chlorite as the chlorine dioxide releasing agent with a strength of 0.25% to 10% (0.5-4.0% nominal), a surfactant (cationic, anionic, non-ionic, amphoteric or any ratio or combination thereof) with an inclusion rate of 0.5 - 25%, and a diluent (preferably water, alternatively glycols, ethers, surfactants or any combination of these) which is deployed via a foam pump apparatus. |
| Wipe | A substrate (synthetic, naturally derived, or any combination, with or without production-included dyes), impregnated with a solution of the above liquid formulation. |

**Table 4: Second part examples**

| **Form** | **Description** |
|---|---|
| Powder | A product containing powdered acid (preferably citric acid anhydrous, however can be substituted for comparable acids such, but not limited to, malic or tartaric acid) with an inclusion rate of 40-90% (nominal 60%), a dyestuff selective for chlorine dioxide, with an inclusion rate of 0.01%-5.00% (nominal 1.00%), and a free flow agent to maintain powder mobility and reduce clumping (preferably fumed silica). |
| Liquid | A product containing a soluble/dissolved acid (preferably citric acid monohydrate, however can be substituted for comparable acids, or combination of, such as, but not limited to, malic, tartaric, citric acid anhydrous, phosphoric, hydrochloric or sulphuric acid) with an inclusion rate of 1-30% (nominal 1-10%), a dyestuff selective for chlorine dioxide, with an inclusion rate of 0.01%-5.00% (nominal 1.00%), and a diluent (preferably water, alternatively glycols, ethers, surfactants or any combination of these). |
| Foam | A product containing a soluble/dissolved acid (preferably citric acid monohydrate, however can be substituted for comparable acids, or combination of, such as, but not limited to, malic, tartaric, citric acid anhydrous, phosphoric, hydrochloric or sulphuric acid) with an inclusion rate of 1-30% (nominal 1-10%), a dyestuff selective for chlorine dioxide, with an inclusion rate of 0.01%-5.00% (nominal 1.00%), a surfactant (cationic, anionic, non-ionic, amphoteric or any ratio or combination thereof) with an inclusion rate of 0.5 - 25%, and a diluent (preferably water, alternatively glycols, ethers, surfactants or any combination of these) which is deployed via a foam pump apparatus. |
| Wipe | A substrate (synthetic, naturally derived, or any combination of these) impregnated with a solution of the above liquid formulation. |
| | The dyestuff selective for chlorine dioxide can be incorporated, if desired, into the manufacturing process of the substrate material, so that it is in-situ prior to impregnation with liquid. |

In either or both parts, additional components may be added to enhance performance or provide desired effects or behaviour. These include, for example, powdered/liquid surfactants (preferably non-ionic, but may include cationic, amphoteric and/or anionic); chelating agents with high affinity for sodium sequestration (to increase rate of sodium chlorite decomposition) and foam building; dyes; fragrances; fragrance suppressants (such as zeolites); secondary oxidisers such as sodium percarbonate; absorbent materials (including superabsorbent polymers, naturally derived clays and pumice blends etc.); and thickening agents.

Table 5 presents some possible combinations of these delivery forms. In some cases, mixing of the parts results in a concentrated disinfecting composition, which may be diluted before use. In other cases, mixing of the parts creates a ready-to-use disinfecting product. The ratio of the parts for mixing can be selected according to the composition and application, and may be between 1:3 and 3:1 or greater. In some examples, the ratio is 1:1.

**Table 5: Possible delivery combinations**

| **First part** | **Second part** | **Further dilution** |
|---|---|---|
| Liquid | Liquid | Dependant on application |
| Liquid | Powder | Dependant on application |
| Liquid | Wipe | No |
| Liquid | Foam | Dependant on application |
| Powder | Powder | Dependant on application |
| Powder | Liquid | Dependant on application |
| Powder | Wipe | No |
| Powder | Foam | Dependant on application |
| Wipe | Liquid | Dependant on application |
| Wipe | Foam | No |
| Foam | Liquid | Dependant on application |
| Foam | Powder | Dependant on application |
| Foam | Wipe | No |
| Foam | Foam | Dependant on application |

A delivery combination in which both the first and second parts are delivered as foams is described in WO 2006/079822 A1. In such a system, the foams can be mixed together after delivery from a dispenser, or may be mixed within the dispenser immediately before delivery. In the context of the present invention, the selective dyestuff is added to either or both of the first and second parts, so that the mixture of foams first exhibits the pre-change colour of the dyestuff, and then the colour changes to the post-oxidation colour once sufficient chlorine dioxide has been generated in the mixture. The mixed foams can be applied directly to a surface or item, to a wipe for subsequent application to the surface, or in any other suitable way.

Further modifications and variations not explicitly described above may also be contemplated without departing from the scope of the invention as defined in the appended claims.

## Claims

1. A disinfectant system comprising:
(a) a first part comprising a first reagent in a carrier medium; and
(b) a second part which is miscible with the first part and which comprises a second reagent in a carrier medium;
wherein the first reagent and the second reagent will react when the first and second parts are mixed to provide a chlorine dioxide disinfecting composition;
**characterised in that**
the first part or the second part further comprises a dyestuff that oxidises in the presence of chlorine dioxide to produce a visible colour change upon mixing of the first part with the second part;
wherein said colour change does not occur upon exposure of the dyestuff-containing part to a hydrogen peroxide disinfectant and/or a peracetic acid disinfectant;
wherein the dyestuff is an anthocyanin dyestuff selected from the group consisting of purple carrot extract, haskapa berry extract, blackcurrant extract, and black carrot extract, or an anthocyanidin dyestuff selected from the group consisting of bilberry extract and blue pea extract, or a betanin dyestuff selected from the group consisting of red beetroot powder and beetroot juice concentrate.

2. A disinfectant system according to Claim 1, wherein the dyestuff changes from coloured to colourless in the presence of chlorine dioxide.

3. A disinfectant system according to Claim 1 or Claim 2, wherein the first reagent comprises a metal chlorite and the second reagent comprises an acid.

4. A disinfectant system according to any of the preceding claims, wherein the first part is contained in a dispenser whereby it may be dispensed as a fluid.

5. A disinfectant system according to any of the preceding claims, wherein the second part is absorbed or impregnated in at least one wipe (16).

6. A disinfectant system according to any of Claims 1 to 4, wherein the first part and the second part each comprise liquids.

7. A disinfectant system according to any of Claim 1 to 4, wherein the first part and the second part each comprise foams.

8. A disinfectant system according to any of the preceding claims, wherein the second part comprises the dyestuff.

9. A disinfectant system according to any of the preceding claims, wherein the first part or the second part comprises between about 0.1 wt% and about 2 wt% of the dyestuff.

## Patentansprüche

1. Desinfektionssystem, umfassend:
(a) einen ersten Teil, welcher ein erstes Reagenz in einem Trägermedium umfasst; und
(b) einen zweiten Teil, welcher mit dem ersten Teil mischbar ist und der ein zweites Reagenz in einem Trägermedium umfasst;
wobei das erste Reagenz und das zweite Reagenz miteinander reagieren, wenn der erste und der zweiten Teil gemischt werden, sodass eine Chlordioxid enthaltende Desinfektionszusammensetzung bereitgestellt wird; **dadurch gekennzeichnet,**
**dass** der erste Teil oder der zweite Teil ferner einen Farbstoff umfasst, der in Gegenwart von Chlordioxid oxidiert, sodass beim Mischen des ersten Teils mit dem zweiten Teil eine sichtbare Farbänderung erzeugt wird; wobei diese Farbänderung nicht auftritt, wenn der den Farbstoff enthaltende Teil einem Wasserstoffperoxid-Desinfektionsmittel und/oder einem Peressigsäure-Desinfektionsmittel ausgesetzt wird;
wobei der Farbstoff
ein Anthocyan-Farbstoff, ausgewählt aus der Gruppe bestehend aus violettem Karottenextrakt, Haskap-Beerenextrakt, schwarzem Johannisbeerextrakt und schwarzem Karottenextrakt,
oder ein Anthocyanidin-Farbstoff, ausgewählt aus der Gruppe bestehend aus Heidelbeerextrakt und Blauerbsen-Extrakt,
oder ein Betanin-Farbstoff, ausgewählt aus der Gruppe bestehend aus rotem Rote-Bete-Pulver und Rote-Bete-Saftkonzentrat ist.

2. Desinfektionssystem nach Anspruch 1,
bei welchem der Farbstoff in Gegenwart von Chlordioxid von farbig nach farblos wechselt.

3. Desinfektionssystem nach Anspruch 1 oder 2,
bei welchem das erste Reagenz ein Metallchlorit und das zweite Reagenz eine Säure umfasst.

4. Desinfektionssystem nach einem der vorigen Ansprüche, bei welchem der erste Teil in einem Spender enthalten ist, wodurch er als Fluid verspendet werden kann.

5. Desinfektionssystem nach einem der vorigen Ansprüche, bei welchem der zweite Teil in wenigstens einem Wischtuch (16) absorbiert oder darin imprägniert ist.

6. Desinfektionssystem nach einem der Ansprüche 1 bis 4, bei welchem der erste Teil und der zweite Teil jeweils Flüssigkeiten umfassen.

7. Desinfektionssystem nach einem der Ansprüche 1 bis 4, bei welchem der erste Teil und der zweite Teil jeweils Schäume umfassen.

8. Desinfektionssystem nach einem der vorigen Ansprüche, bei welchem der zweite Teil den Farbstoff umfasst.

9. Desinfektionssystem nach einem der vorigen Ansprüche, bei welchem der erste Teil oder der zweite Teil zwischen etwa 0,1 Gew.-% und etwa 2 Gew.-% von dem Farbstoff umfasst.

## Revendications

1. - Système désinfectant comprenant :
(a) une première partie comprenant un premier réactif dans un milieu de support; et
(b) une seconde partie qui est miscible avec la première partie et qui comprend un second réactif dans un milieu de support ;
dans lequel le premier réactif et le second réactif réagiront lorsque les première et seconde parties seront mélangées pour fournir une composition désinfectante au dioxyde de chlore ;
**caractérisé par le fait que**
la première partie ou la seconde partie comprend en outre un colorant qui s'oxyde en présence de dioxyde de chlore pour produire un changement de couleur visible lors du mélange de la première partie avec la seconde partie ;
dans lequel ledit changement de couleur ne se produit pas lors de l'exposition de la partie contenant un colorant à un désinfectant au peroxyde d'hydrogène et/ou à un désinfectant à l'acide peracétique ;
dans lequel le colorant est un colorant d'anthocyanine choisi dans le groupe constitué par un extrait de carotte pourpre, un extrait de baie de haskap, un extrait de cassis et un extrait de carotte noire, ou un colorant d'anthocyanidine choisi dans le groupe constitué par un extrait de myrtille et un extrait de pois bleu, ou un colorant de bétanine choisi dans le groupe constitué par une poudre de betterave rouge et un concentré de jus de betterave.

2. - Système désinfectant selon la revendication 1, dans lequel le colorant passe de coloré à incolore en présence de dioxyde de chlore.

3. - Système désinfectant selon la revendication 1 ou la revendication 2, dans lequel le premier réactif comprend un chlorite métallique et le second réactif comprend un acide.

4. - Système désinfectant selon l'une quelconque des revendications précédentes, dans lequel la première partie est contenue dans un distributeur, ce par quoi elle peut être distribuée en tant que fluide.

5. - Système désinfectant selon l'une quelconque des revendications précédentes, dans lequel la seconde partie est absorbée ou imprégnée dans au moins une lingette (16).

6. - Système désinfectant selon l'une quelconque des revendications 1 à 4, dans lequel la première partie et la seconde partie comprennent chacune des liquides.

7. - Système désinfectant selon l'une quelconque des revendications 1 à 4, dans lequel la première partie et la seconde partie comprennent chacune des mousses.

8. - Système désinfectant selon l'une quelconque des revendications précédentes, dans lequel la seconde partie comprend le colorant.

9. - Système désinfectant selon l'une quelconque des revendications précédentes, dans lequel la première partie ou la seconde partie comprend entre environ 0,1 % en poids et environ 2 % en poids du colorant.
